# EUROPEAN PATENT APPLICATION

(11) **EP 1 319 407 A1**
(43) Date of publication of application: **18.06.2003**
(21) Application number: 01811228.4
(22) Date of filing: 14.12.2001
(51) Int. Cl.: A61K 35/20, A61K 7/48, A61K 9/107, A61K 45/06, A61P 17/00

(54) **Pharmaceutical composition for topical treatment of skin disorders and skin wounds**

(71) Applicant: Montoie Import-Export S.A., 1007 Lausanne (CH)
(72) Inventor: Fardoussi, Kasem Khal, Alep (SY)
(74) Representative: Ganguillet, Cyril

(57) **Abstract**

Use of milk fat globules for the manufacture of a pharmaceutical composition intended for the topical treatment of skin disorders and skin wounds; the composition may further contain zinc oxide.

## Description

The present invention concerns pharmaceutical compositions intended for a topical treatment of skin disorders and skin wounds. Among the targeted disorders are various inflammatory skin diseases like sheilitis, skin hypercornification, dry dermatitis, skin fissuring, black hyperpigmentation around the eyes and different body surfaces, skin maceration or comedones.

The invention concerns in particular a pharmaceutical composition intended for the topical treatment of acne vulgaris. Acne is a very common disease around the world and acne patients often suffer from a psychological disturbance because of the apparent lesions on their skin. Very often this condition persists for a long time and some severe acne lesions may cause skin distortion of the face. Medications against acne available in the art are of limited efficiency, with side effects such as redness, irritation, burning and peeling of skin and with recurrence of lesions sometimes after the stop of the medication. Because of that, acne still needs a new medication that could cure acne lesions without side effects.

It has often been observed that persons suffering from acne also exhibit a greasy skin and an accumulation of subcutaneous skin fat. Therefore another object of the present invention is to propose a pharmaceutical composition for decreasing subcutaneous skin fat and skin sliminess.

Suffering from burns as a result of direct contact with boiling water, hot oil, hot objects, chemicals like acids, fires and various related forms of energy such as light rays, ultraviolet rays, roentgen rays, radiations from radioactive substance and electric current are very common. All these blows tend to coagulate cell protoplasm, thus killing the cell and if sufficiently severe, to carbonise it. Burns are divided into three categories according to severity of injury:
1. First degree burns (superficial burns) are the milder burns, which may not immediately kill even the epidermis, but cause erythema. The epidermis usually desquamates week later, as in the case of sunburns and mild burns by ultraviolet light.
2. Second degree burns (partial thickness) also produce a serious inflammatory exudate in the form of vesicles (blisters) between the epidermis and the dermis.
3. Third degree burns (complete skin thickness) kill the tissue outright to an appreciable depth and sometimes deep down, involving subcutaneous tissue and muscles.

Injuries as a result of second and third degrees burns cause excruciating and often unbearable pain, especially when the burn is superficial. Strong doses of painkillers are usually prescribed to ease suffering. In addition, patients must endure a long-time agony waiting for injuries to heal. But what patients fear most and ask about immediately after being injured are the everlasting skin scars, particularly in the face, hands and feet.

Accordingly, there exists a strong need for pharmaceutical compositions enhancing skin repair after burns.

The above objectives are achieved by using milk fat globules for the manufacture of a pharmaceutical composition intended for the topical treatment of skin disorders and skin wounds.

Milk fat globules (MFG) consist at 99 % in weight of a triglyceride core. This core is surrounded by a lipid bilayer containing integral membrane proteins (Mather and Keenan, J. Membrane Biol. 21, 65, 1975). The globules have a size range from 1 to 10 µm diameter and are surrounded by a thin membrane called the milk fat globule membrane (MFGM). This membrane, which is approximately 10 nm thick in cross-section, consists of a complex mixture of proteins, glycoproteins and enzymes, phospholipids, triglycerides, cholesterol and other minor components (McPherson et al., J. Dairy Research 50, 107, 1983). Studies of the membrane structure have revealed that proteins, and in particular glycoproteins, are arranged in the membrane so that portions of the protein chains are exposed on the external surface of the MFGM (see for example Kobylka et al., Biochim. Biophys. Acta 288, 282, 1972).

The triglyceride core comprises polysaturated and polyunsaturated fatty acids, attached to glycerine groups. The most important polyunsaturated fatty acids are oleic, linolenic, arachidonic and in particular linoleic acids. These fatty acids have anti-inflammatory properties. It has been reported that the rise in acne lesions upon puberty is often accompanied by a local decrease of linoleic acid in epidermal and comedonal lipids, related to ductal hypercornification (Kellum, R.L., Strangfeld, K.E., J. Invest. Dermatol 58, 315, 1972). The inventor therefore believes that in one aspect, the pharmaceutical composition according to the invention supplies linoleic acid and consequently corrects ductal hypercornification of acne suffering patients.

Pilosebaceous units comprise a holocrine acinar gland embedded in the dermis, each gland opening into a short duct, which ends in the upper portion of the hair follicle. Acinar cells proliferate and differentiate and are gradually filled with sebum droplets and burst. The sebum is composed of a mixture of lipids comprising triglycerides, free fatty acids, cholesterol and its esters. The flow of sebaceous glands is normally continuous and a disturbance in the normal secretion and flow of sebum is one of the causes favouring colonisation of propionibacterium acnes and development of acne. Studies of interaction of propionibacterium acnes with skin lipids have shown that free fatty acids and bacterial lipase may be necessary for clumping and ductal colonisation (Gribbon, E. M. et al., J. Gen. Microbiol. 139, 1745, 1993). MFGs, due to their liposomal structure, have the ability to penetrate skin layers, to reach and to concentrate in the pilosebaceous units. In the pilosebaceous units, the MFGs are capable to impair the action of bacterial lipase enzymes, in particular to impair a lipase of propionibacterium acnes from breaking down lipids extrated from sebaceous glands into free fatty acids. MFGs are also capable to form an emulsion with previously accumulated free fatty acids and possibly may facilitate the passage of the emulsion into nearby lymph vessels. It is widely accepted that free fatty acids are skin irritant. Therefore, it is believed that MFGs prevent an inflammatory process associated with the irritating effect of free fatty acids in the skin.

Preferably, the milk fat globules are incorporated in the pharmaceutical composition of the invention as an oil-in-water emulsion. Thereby, their liposomal structure and ability to emulsify free fatty acids is maintained. For avoiding disruption of milk fat globules, aggregation of the same and/or loss of membrane components, the extraction process of the milk fat globules from the raw starting material, namely milk, is carried out under gentle conditions, avoiding especially too high temperatures and excessive centrifugation speeds. Sheep milk was found to be a preferred starting material for preparing the pharmaceutical composition of the invention.

Preferably, the amount of said milk fat globules is between 7 % and 70 % of the weight of said composition.

According to a preferred embodiment of the invention, the pharmaceutical composition further contains zinc oxide. This agent has the property of decreasing congestion and absorption of inflammatory products. It is also known that zinc oxide is a mild astringent for the skin and has a smoothing, protective and antiseptic action. The inventor found that zinc oxide and MFGs have a synergistic effect in the composition of the invention.

Conveniently, the total amount of milk fat globules and zinc oxide is between 7 % and 70 % by weight of said composition.

The pharmaceutical composition of the invention, based on the use of MFGs, may incorporate, instead of zinc oxide or in addition to the latter, either an antibiotic or a bactericidal drug or a sulphonamide or a retinoid. The pharmaceutical composition of the invention may incorporate further various additives, preservatives or antioxidants known in the gallenic art.

The process for making the pharmaceutical composition of the invention and the properties of the same will be better understood by those skilled in the art in view of the following description of two formulations, given by way of examples and by the results of clinical tests performed with the same.

### Example 1: isolation of milk fat globules from sheep milk

The milk fat globules are obtained from raw ruminants milk by centrifugation at 1500 rotations/minute for ten minutes at room temperature followed by removal of the sub-natant aqueous phase. This process is repeated three times at 60° C after resuspending the MFG in water. The intermediate oily material obtained after centrifugation may contain up to 95 - 97 % MFGs. This product is further processed to the pharmaceutical composition of the invention in form of an oil-in-water emulsion.

### Example 2: cream incorporating MFG and zinc oxide

The formula of the cream is the following:

| | |
|---|---|
| MFG | 8 % |
| Zinc oxide (Smith glass) | 8 % |
| Cetearic acid | 7 % |
| Tri-ethanol amine (basf) | 1.5 % |
| Paraffin liquid | 10 % |
| Vasilen | 2 % |
| * Phinova (Croda chemicals) | 0.5 % |
| Distilled water | q.s. 100% % |

| | |
|---|---|
| * Phinova is an antioxidant and a preservative agent, to prevent bacterial contamination, consisting of methyl parabin, propyl parabin and PHT. | |

The cream is prepared as follows:
1. The water is put in a mixer at a speed of 1250 rotations/minute, heated up to 75° C. At this stage, the materials, which are able to melt in water, are added.
2. The oily materials are placed into a mixer at an ordinary speed, approximately for 10 - 15 min., and heated up to 70° C, which is considered to be the melting point.
3. Zinc oxide is slowly added to water during the mixing which is continued during four minutes.
4. The melted oily materials are slowly added to the mixture of water and zinc oxide. The mixing is continued at a speed of 1250 rotations/minute up to 10 minutes and then the speed of mixing is reduced to slow speed.
5. The cream is passed several times from the mixer through a homogeniser.
6. The cream is slowly chilled until its temperature is suitable for filling and during this, is passed several times into the homogeniser.
7. The pH of the cream is adjusted to 7.5 - 8.
8. The cream is withdrawn from the mixer, passed to the homogeniser, then to the filling machine.
9. The cream is stored between 20 and 25° C.

### Example 3: evaluation of emulsion stability

Emulsion stability may be assessed after storage of the product for proposed shelf-life; this, however, is timeconsuming and most workers rely on accelerated tests to provide information of long-term stability. Accelerated tests involve placing emulsion under stress; this is normally applied using temperature manipulation techniques, which is the most commonly employed type of accelerated stability tests. This test was done by putting the cream in a temperature of -15° C for 24 hours. The cream then was directly withdrawn and placed in an incubator at 45° C for 24 hours. This procedure was repeated six times without any changes in the cream emulsion stability. It is estimated that the half shelf-life of the cream is approximately twelve months and that the final cream could be used for up to one year of manufacturing.

### Example 4: acne treatment

The formulation of example 2 was tested on a group of volunteers exhibiting acne pimples.

### a. The trial group

Before entering the real trial, the cream was applied to a random sample of volunteers for a period of time ranging from one to six months, to ensure the safety of application procedure. In the real trial, the cream has been used on 143 volunteers suffering from different degrees of acne without any diet regime. 90 patients continued the trial up to 180 days. Almost all these patients had previously used different medications with limited improvement and recurrence of the acne.

Statistical analysis was done on the 90 patients who continued the trial. The patients were also classified according to their marital status. Married patients were 18.9 % and single patients were 81.1 %. The patients were classified according to their sex and severity of acne lesions, which ranged from mild to moderate or sever (Table 1). 54 patients were males and 36 patients were females. The patients' age ranged between 15 - 35 years. The whole group was clinically evaluated.

**Table 1:**

| The relation between the severity of acne lesions and the sex of 90 patients. | | | | |
|---|---|---|---|---|
| **Severity of acne lesion (% of 90)** | | | | |
| | Mild | Moderate | Severe | Total |
| Male | 18.9 | 20.0 | 21.1 | 60.0 |
| Female | 12.2 | 17.8 | 10.0 | 40.0 |
| Total | 31.1 | 37.8 | 31.1 | 100.0 |

### b. Application schedule

1. The cream is applied twice a day and kept at room temperature.
2. The area of the lesions is washed with soap and warm water before using the cream.
3. It is recommended not to expose to sunlight for at least one hour after having applied the cream.
4. The cream is applied until the affected area becomes as normal as the surrounding unaffected skin.
5. The volunteers were asked not to use any other medication during the trial.

### c. Results

It was noticed in many of the group trial volunteers a fast improvement which was obvious during the first three weeks after using the cream. After this period, the improvement slowed down gradually until the complete cure of all forms of acne which were present before the treatment. It was also noticed in all treated patients absence of the oily appearance of the facial skin during the treatment period.

Some patients suffered from a temporarily appearance of new acne lesions in places where the acne was not present previously between two to six weeks after application of the cream. For these patients the improvement started again gradually towards a complete recovery. In very few cases, it was also noticed an acceptable light redness in the area where the cream was applied, especially in patients who have white skin. The skin redness disappeared with the continuation of the treatment. A slight temporarily skin exfoliation was also seen in very few patients.

It is obvious from table 2 that curability between 50 and 100 % was observed on 90 % of the patients. Most of these patients received a treatment for a period of time ranged from 90 to 150 days. On the other hand, less than 50 % of the cure was seen in 10 % of the patients who used the preparation from 90 to 150 days. 10 % of these patients were either partially cured or not cured at all. It is also found that there is no correlation between the sex and acne lesion curability and between the different colours of the skin.

**Table 2:**

| relation between the periods of treatment and the cure percentage of 90 patients. | | | | |
|---|---|---|---|---|
| **Period of treatment (day)** | | | | |
| Cure % | 90 - 120 | 121 - 150 | 151 - 180 | Total |
| 100 | 21.1 | 16.7 | * | 37.8 |
| 76 - 90 | 25.6 | 15.6 | 2.2 | 43.3 |
| 50 - 75 | 2.2 | 4.4 | 2.2 | 8.9 |
| Less than 50 | 3.3 | 6.7 | - | 10.0 |
| Total | 52.2 % | 43.3 % | 4.4 % | 100.0 % |

| | | | | |
|---|---|---|---|---|
| * Patient were completely cured at less than 150 days of treatment | | | | |

The fast improvement of acne lesions indicates that the cream ingredients used in this trial were effective directly on the lesions without a delay. However, the slowing down of the improvement was probably due to the time needed for complete recovery, repair the cornfield follicles and getting red of the accumulated debris in the pilosebaceous units of the skin. It is suggested that the absence of the oily appearance of the skin during the treatment course is because the impairment of the lipase enzyme in the sebaceous glands. This impairment could be due to the prevention of the lipase enzyme from withdrawing more fatty acids from blood to the sebaceous glands and decreasing the synthesis of new sebocytes.

The temporarily appearance of a new acne lesions during the first few weeks of therapy was probably due to the action of the medication on deep, previously unseen pimples. The appearance of new pimples should not be considered as a reason to discontinue the medication and an improvement will start again gradually.

The slight redness and peeling that was experienced by a very few patients is manageable and diminishes over two weeks. This observation could be related to the nature of the skin in white colour patients. The slight temporarily skin peeling may be an associated with the repaired skin and removal of died skin cells.

### Example 5: treatment of skin burns

The cream of example 2 was tested on a group of patients suffering from burns of various degrees.

After receiving the first aid, patients have been clinically evaluated to assess the burn degree and the presence of complication or contamination. 247 volunteers were enrolled in this trial ranging in age from 2 to 65 years old including males and females. All trial group patients suffered from first to third degree burns. The third degree burns in trial involve only the epidermis and dermis. The causes of burns include boiling liquids, boiling oils and fires. The affected area ranged from 5 to 25 % of the body surface. The treated burns include faces, arms, legs, chests, abdomens, backs and genital areas. The cream was used alone without any other topical medication or disinfectant.

The patients in this trial were classified depending on the following degrees of burns:

| No. of patients | Degree of burns |
|---|---|
| 134 | First degree |
| 75 | Second degree |
| 38 | Third degree* |

| | |
|---|---|
| *Third degree burns include the injury of the epidermis anddermis only | |

In the first degree burns, the preparation is first applied to the burned area as a layer of 0.2 mm thickness every eight hours for an average of five days and a complete cure is ensured. In the second and third degree burns, the preparation is first applied to the burned area as a layer of 0.2 mm thickness and wrapped with sterile gauze. This method of application is repeated every 24 hours. If the burned area could not be wrapped with gauze, the preparation was applied every eight hours. The use of preparation was continued until the affected area became as normal as the adjacent skin area. This may take from two to four weeks, depending on the burn degree, the cause and the presence of other complications. After a partial cure, the patient receives the preparation without the gauze each eight hours. In the case the affected area was around joints or in a folded skin as in the neck area, the burned area was kept open to prevent skin adhesions.

### The results:

In this trial, the cure of patients having the first and second degrees burns was a hundred percent. The affected skin returned to normally appearing skin as the adjacent unaffected area. The cure of patients with the third degree burns were complete unless there was a delay in the treatment or complications. In this case, the cure may reach up to 85 %. The optimum results could be obtained when the preparation is used immediately after the patient has received the first aid and before contamination and other complications occur.

### Example 6: composition for decreasing locally accumulated subcutaneous fat

A cream is formulated with the following composition:

| | |
|---|---|
| MFG | 7 % |
| Cucumber seed oil | 1 % |
| Cetearic acid | 7 % |
| Tri-ethanol amine | 1.5 % |
| Paraffin liquid | 7 % |
| Paraffin hard | 1.5 % |
| Honey | 1.5 % |
| Isopropyl myristate | 6 % |
| Phinova | 0.5 % |
| Distilled water | q.s. 100 % |

The preparation of the cream is similar to the preparation described in examples 1 and 2, except that zinc oxide is not present. The evaluation of the emulsion stability leads to results showing that the stability is closely similar to the emulsion of example 2.

### Example 7: treatment of local subcutaneous fat accumulations

The trial was done on 200 volunteers. Half of them were married and their age ranged between 20 and 50 years old. The preparation was applied on different areas of the volunteers' body, including abdomen, pelvis area and thighs. All volunteers were asked to follow a slight diet during the experiment. The results obtained indicated a decrease of the accumulated subcutaneous fat between 70 and 90 %. This depends on the area that was treated and the duration of the application. It was noticed that the area exhibiting the fastest response to the preparation was the abdominal area, especially in women after parturition, for unknown reasons. It was noted that the preparation is useless if the patient is greedy and does not follow at least a slight diet. During the trial, 5 % of the volunteers suffered from local skin hypersensitivity, indicating that the application should be immediately stopped if any sign of skin irritation appears. The preparation is also not recommended to be used by persons suffering from leg lymphangitis or varicose veins, since a few persons of this trial, affected by these conditions, complained of leg pain.

## Claims

1. The use of milk fat globules for the manufacture of a pharmaceutical composition intended for the topical treatment of skin disorders and skin wounds.

2. Use as claimed in claim 1, **characterised in that** said pharmaceutical composition is an oil-in-water emulsion of milk fat globules.

3. Use as claimed in claim 2, **characterised in that** the amount of said milk fat globules is between 7 % and 70 % by weight of said composition.

4. Use as claimed in anyone of the preceding claims, **characterised in that** said pharmaceutical composition further contains zinc oxide.

5. Use as claimed in claim 4, **characterised in that** the total amount of milk fat globules and zinc oxide is between 7 % and 70 % by weight of said composition and **in that** said amount of milk fat globules is not less than 7 % by weight of said composition.

6. Use as claimed in anyone of the preceding claims, **characterised in that** said composition further comprises an antibiotic, a bactericidal drug, a sulphonamide or a retinoid.

7. Use as claimed in anyone of the preceding claims, for the manufacture of a pharmaceutical composition for the treatment of a disorder selected from acne vulgaris, comedones, black hyperpigmentation, maceration, skin fissuring, dry dermatitis, hypercornification, sheilitis.

8. Use as claimed in anyone of claims 1 to 6 for the manufacture of a pharmaceutical composition for the treatment of skin burns.

9. Use as claimed in anyone of claims 1 to 6 for the manufacture of a composition for decreasing subcutaneous skin fat or skin sliminess.
